# EUROPEAN PATENT APPLICATION

(11) **EP 4 552 519 A1**
(43) Date of publication of application: **14.05.2025**
(21) Application number: 22951141.5
(22) Date of filing: 14.07.2022
(51) Int. Cl.: A24F 40/51, A24F 40/20

(54) **INHALATION DEVICE AND CONTROL METHOD**

(71) Applicant: Japan Tobacco Inc., Tokyo 105-6927 (JP)
(72) Inventor: FUJIKI, Takashi, Tokyo 130-8603 (JP)
(74) Representative: Hoffmann Eitle
(86) International application number: PCT/JP2022/027689
(87) International publication number: WO 2024/013927

(57) **Abstract**

[Problem] To accurately determine the state of an accommodating part that can accommodate a stick-type base material in an inhalation device.

[Solution] This inhalation device comprises: an accommodating part that can accommodate a stick-type base material in an internal space; a first detection unit that emits light into the internal space and detects received reflected light; a second detection unit that detects the motion of a user relative to the inhalation device; a control unit that makes a determination regarding the state of the accommodating part on the basis of a first detection value corresponding to the intensity of the reflected light detected by the first detection unit; and a storage unit that stores a correction value used to calculate the first detection value by correcting a second detection value indicating the intensity of the reflected light detected by the first detection unit. The control unit controls a process of calculating the correction value and storing the correction value in the storage unit when a prescribed detection result is obtained by the second detection unit.

## Description

### Technical Field

The present invention relates to an inhaler device and a control method.

### Background Art

An inhaler device that generates material to be inhaled by a user, such as an electronic tobacco and a nebulizer, is widely used. For example, an inhaler device uses a substrate including an aerosol source for generating an aerosol, a flavor source for imparting a flavor component to the generated aerosol, and the like, to generate an aerosol with the imparted flavor component. The user is able to taste a flavor by inhaling the aerosol with the imparted flavor component, generated by the inhaler device.

With the aim of further improving the quality of user experience at the time of using such an inhaler device, various technologies are under development. For example, PTL 1 describes a technology to radiate light, detect the phosphorescent characteristics of reflected light, and control the operation of an inhaler device in accordance with a detection result.

### Citation List

### Patent Literature

Patent Literature 1: JP 2019-528710 A

### Summary of Invention

### Technical Problem

A user is able to use the inhaler device when the user accommodates a stick substrate including an aerosol source in a container. For this reason, the inhaler device is capable of appropriately control the operation of the inhaler device by determining the state of the container. Thus, improvement in the accuracy of determination on the state of the container is desired.

The present invention is contemplated in view of the above problem, and it is an object of the present invention to provide a new, improved inhaler device and a new, improved control method that are capable of accurately determining a state of a container.

### Solution to Problem

To solve the above problem, one aspect of the present invention provides an inhaler device. The inhaler device includes a container capable of accommodating a stick substrate in an internal space, a first detector that radiates light to the internal space and detects reflected light received, a second detector that detects an action of a user to the inhaler device, a controller that performs a determination on a state of the controller in accordance with a first detection value corresponding to an intensity of reflected light detected by the first detector, and a memory that stores a correction value used to calculate the first detection value by correcting a second detection value indicating an intensity of reflected light detected by the first detector. The controller controls a process of, when a predetermined detection result is obtained by the second detector, calculating the correction value and storing the correction value in the memory.

The correction value may be a difference value between a predetermined detection reference value and the second detection value output from the first detector, the first detection value may be a value obtained by subtracting the correction value from the second detection value, and the controller may control a process of, when a predetermined detection result is obtained by the second detector, calculating the first detection value.

The controller may control to switch a mode of the first detector to an operation mode in which the reflected light is detected or a sleep mode in which detection of the reflected light is stopped in accordance with a detection result obtained by the second detector.

The inhaler device may further include a lid capable of opening and closing an opening that communicates with the internal space of the container, and the second detector may detect opening and closing of the opening with the lid.

The controller may control to switch a mode of the first detector to the operation mode when opening of the opening with the lid is detected by the second detector and control to switch the mode of the first detector to the sleep mode when closing of the opening with the lid is detected by the second detector.

The controller may control a process of calculating the correction value when opening of the opening with the lid is detected by the second detector.

The controller may control a process of calculating the correction value and storing the calculated correction value in the memory when closing of the opening with the lid is detected by the second detector and control a process of correcting the second detection value by using the correction value stored in the memory when the mode of the first detector is switched to the operation mode after the storage.

The inhaler device may further include a power supply that stores electric power, and the second detector may detect connection and cancellation of charging of the power supply by the user.

The controller may control to switch the mode of the first detector to the operation mode when cancellation of charging of the power supply is detected by the second detector and to switch the mode of the first detector to the sleep mode when connection of charging of the power supply is detected by the second detector.

The controller may calculate the correction value when cancellation of charging of the power supply is detected by the second detector.

The controller may control a process of calculating the correction value and storing the calculated correction value in the memory when connection of charging of the power supply is detected by the second detector and control a process of correcting the second detection value by using the correction value stored in the memory when the mode of the first detector is switched to the operation mode after the storage.

The controller may determine whether the stick substrate is accommodated in the internal space in accordance with the first detection value.

The controller may perform a determination on a crud adhering to the container in accordance with the first detection value.

The controller may perform a determination on a state of the container in accordance with the first detection value and a predetermined threshold and correct the threshold in accordance with a temporal change in the correction value.

To solve the above problem, another aspect of the present invention provides a control method for an inhaler device. The control method is executed by a computer. The inhaler device includes a container capable of accommodating a stick substrate in an internal space, a first detector that radiates light to the internal space and detects reflected light received, a second detector that detects an action of a user to the inhaler device, and a memory that stores a correction value used to calculate a first detection value corresponding to an intensity of reflected light detected by the first detector by correcting a second detection value indicating an intensity of reflected light detected by the first detector. The control method includes performing a determination on a state of the container in accordance with the first detection value, and controlling a process of, when a predetermined detection result is obtained by the second detector, calculating the correction value and storing the correction value in the memory.

### Advantageous Effects of Invention

As described above, according to the present invention, it is possible to accurately determine a state of a container.

### Brief Description of Drawings

[Fig. 1] Fig. 1 is a schematic diagram that schematically illustrates an internal configuration example of an inhaler device.
[Fig. 2] Fig. 2 is an overall perspective view of the inhaler device 100 according to an embodiment.
[Fig. 3] Fig. 3 is an overall perspective view of the inhaler device 100 according to the present embodiment in a state where a stick substrate 150 is held.
[Fig. 4] Fig. 4 is a schematic diagram that illustrates the configuration of the inhaler device 100 according to the present embodiment around a container 140 and the configuration of the stick substrate 150.
[Fig. 5] Fig. 5 is a schematic diagram that illustrates the configuration of the inhaler device 100 according to the present embodiment around the container 140 in detail.
[Fig. 6] Fig. 6 is a schematic diagram of the container 140 of the inhaler device 100 according to the present embodiment when viewed from an opening 142 side.
[Fig. 7] Fig. 7 is a block diagram that illustrates a functional configuration related to detection with an optical sensor 170 in the inhaler device 100 according to the present embodiment.
[Fig. 8] Fig. 8 is a timing chart that illustrates a specific example of detection with the optical sensor 170.
[Fig. 9] Fig. 9 is a view that illustrates a specific example of correction of a second detection value with a detection controller 179.
[Fig. 10] Fig. 10 is a view that illustrates a specific example of determination on a state of the container 140 using a first detection value by a controller 116.
[Fig. 11] Fig. 11 is a view that illustrates a specific example of correction of a second detection value with the detection controller 179.
[Fig. 12] Fig. 12 is a view that illustrates a specific example of determination on a state of the container 140 using a first detection value by the controller 116.
[Fig. 13] Fig. 13 is a view that illustrates a specific example of correction of a first threshold and a second threshold with the detection controller 179.
[Fig. 14] Fig. 14 is a view that illustrates a specific example of determination on a state of the container 140 using a first detection value by the controller 116.
[Fig. 15] Fig. 15 is a view that illustrates a specific example of correction of a second detection value after correction of the first threshold and the second threshold, illustrated with reference to Fig. 13.
[Fig. 16] Fig. 16 is a view that illustrates a specific example of determination on a state of the container 140 using a first detection value by the controller 116.
[Fig. 17] Fig. 17 is a flowchart that illustrates operation control of the inhaler device 100 in accordance with a detection result with the optical sensor 170.
[Fig. 18] Fig. 18 is a flowchart that illustrates the flow of correction of a second detection value with the detection controller 179.

### Description of Embodiments

Hereinafter, a preferred embodiment of the present invention will be described in detail with reference to the attached drawings. In the specification and the drawings, like reference signs denote structural elements having substantially the same functional components, and the description will not be repeated.

### 1. Configuration example of inhaler device

### (1) Internal configuration example

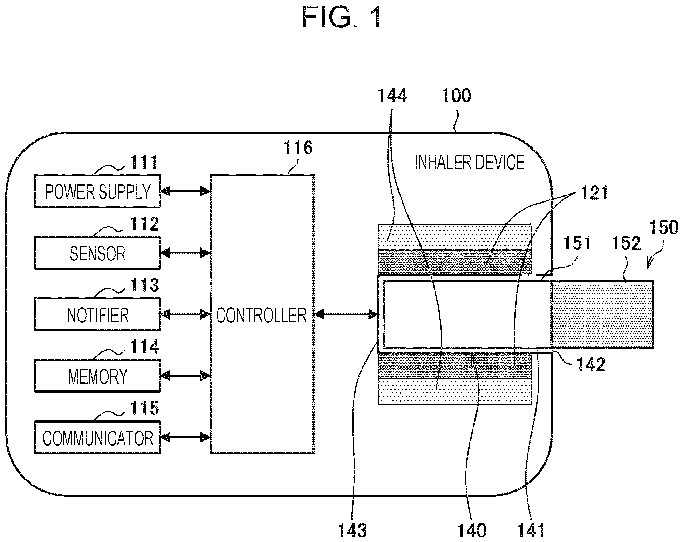

Fig. 1 is a schematic diagram that schematically illustrates an internal configuration example of an inhaler device. As illustrated in Fig. 1, an inhaler device 100 according to the present configuration example includes a power supply 111, a sensor 112, a notifier 113, a memory 114, a communicator 115, a controller 116, a heater 121, a container 140, and a heat insulator 144.

The power supply 111 stores electric power. The power supply 111 supplies electric power to the structural elements of the inhaler device 100 under the control of the controller 116. The power supply 111 can be a rechargeable battery, such as a lithium ion secondary battery.

The sensor 112 acquires various items of information regarding the inhaler device 100. In an example, the sensor 112 may be a pressure sensor such as a capacitor microphone, a flow sensor, or a temperature sensor, and acquire a value generated in accordance with the user's inhalation. In another example, the sensor 112 is an input device that receives information input by the user, such as a button and a switch. The sensor 112 may detect the action of the user. For example, the sensor 112 may detect connection of charging to and disconnection of charging from the power supply 111 by the user. The sensor 112 may detect opening and closing of an opening 142 with a lid 14.

The notifier 113 notifies the user of information. The notifier 113 may be a light-emitting device that emits light, a display device that displays an image, a sound output device that outputs sound, or a vibration device that vibrates.

The memory 114 stores various pieces of information for the operation of the inhaler device 100. The memory 114 is, for example, a non-volatile storage medium, such as a flash memory.

The communicator 115 is a communication interface capable of communication in conformity with any wired or wireless communication standard. For example, a standard using Wi-Fi (registered trademark), Bluetooth (registered trademark), Bluetooth Low Energy (BLE) (registered trademark), near field communication (NFC), or a low-power wide area (LPWA) can be adopted as such a communication standard.

The controller 116 functions as an arithmetic processing unit and a control device and controls the overall operations in the inhaler device 100 in accordance with various programs. The controller 116 is implemented by, for example, an electronic circuit, such as a central processing unit (CPU) and a microprocessor.

The container 140 has an internal space 141. The container 140 holds the stick substrate 150 while accommodating part of the stick substrate 150 in the internal space 141. The container 140 has the opening 142 that allows the internal space 141 to communicate with outside. The container 140 accommodates the stick substrate 150 that is inserted into the internal space 141 through the opening 142. For example, the container 140 is a tubular body having the opening 142 and a bottom 143 at its ends, and defines the pillar-shaped internal space 141. An airflow path for supplying air to the internal space 141 is connected to the container 140. An air inlet hole that is an inlet for air into the airflow path is disposed at, for example, the side of the inhaler device 100. An air outlet hole that is an outlet for air from the airflow path to the internal space 141 is disposed at, for example, the bottom 143.

The stick substrate 150 includes a substrate 151 and an inhalation port 152. The substrate 151 includes an aerosol source. The aerosol source includes a flavor component that is either derived from tobacco or not derived from tobacco. For the inhaler device 100 that is a medical inhaler such as a nebulizer, the aerosol source may include a medicine. The aerosol source may be, for example, a liquid, such as polyhydric alcohol and water, containing a flavor component derived from tobacco or not derived from tobacco. Examples of the polyhydric alcohol include glycerin and propylene glycol. The aerosol source may also be a solid containing a flavor component derived from tobacco or not derived from tobacco. The stick substrate 150 held by the container 140 includes the substrate 151 at least partially accommodated in the internal space 141 and the inhalation port 152 at least partially protruding from the opening 142. When a user inhales with the inhalation port 152 protruding from the opening 142 in his/her mouth, air flows into the internal space 141 through the airflow path (not illustrated), and the air and an aerosol generated from the substrate 151 reach the inside of the mouth of the user.

The heater 121 heats the aerosol source to atomize the aerosol source and generate the aerosol. In the example illustrated in Fig. 1, the heater 121 has a film-like shape and surrounds the outer circumference of the container 140. Subsequently, heat produced from the heater 121 heats the substrate 151 of the stick substrate 150 from the outer circumference, and an aerosol is generated. The heater 121 produces heat when receiving electric power from the power supply 111. In an example, the electric power may be supplied in response to the sensor 112 detecting a start of the user's inhalation and/or an input of predetermined information. Subsequently, the supply of the electric power may be stopped in response to the sensor 112 detecting an end of the user's inhalation and/or an input of predetermined information.

The heat insulator 144 prevents heat from transferring from the heater 121 to the other structural elements. For example, the heat insulator 144 may be a vacuum heat insulator or an aerogel heat insulator.

The configuration example of the inhaler device 100 has been described above. The inhaler device 100 is not limited to the above configuration, and may be configured in various ways as exemplified below.

In an example, the heater 121 may have a blade-like shape, and may be disposed such that the heater 121 protrudes from the bottom 143 of the container 140 toward the internal space 141. In this case, the heater 121 having the blade-like shape is inserted into the substrate 151 of the stick substrate 150 and heats the substrate 151 of the stick substrate 150 from its inside. In another example, the heater 121 may be disposed such that the heater 121 covers the bottom 143 of the container 140. In still another example, the heater 121 may be implemented as a combination of two or more selected from a first heater that covers the outer circumference of the container 140, a second heater having the blade-like shape, and a third heater that covers the bottom 143 of the container 140.

In another example, the container 140 may include an opening/closing mechanism that at least partially opens and closes an outer shell defining the internal space 141. Examples of the opening/closing mechanism include a hinge. In addition, the container 140 may accommodate the stick substrate 150 inserted into the internal space 141 while sandwiching the stick substrate 150, by opening and closing the outer shell. In this case, the heater 121 may be at the sandwiching position of the container 140 and may produce heat while pressing the stick substrate 150.

In addition, means for atomizing the aerosol source is not limited to heating by the heater 121. For example, the means for atomizing the aerosol source may be induction heating.

The inhaler device 100 and the stick substrate 150 may be regarded as making up an aerosol generating system to cooperatively generate an aerosol. Alternatively, the inhaler device 100 may be regarded as including the stick substrate 150.

### (2) External configuration example

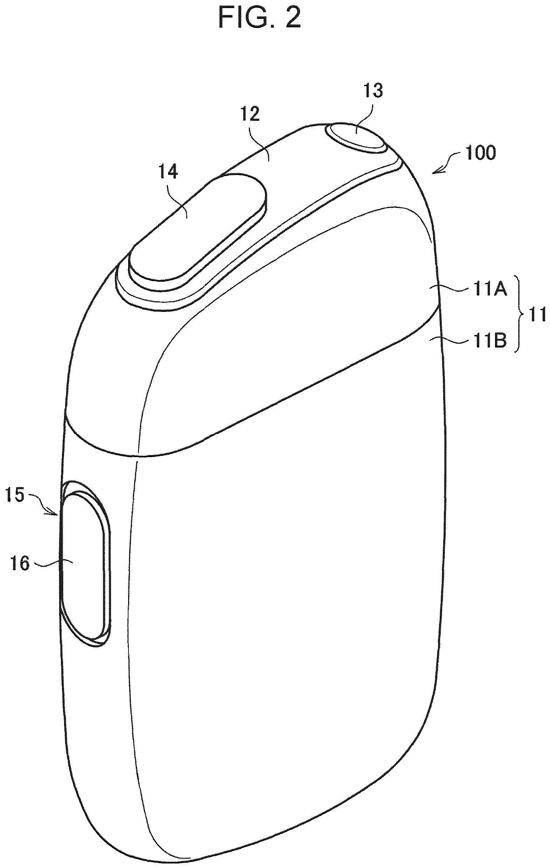

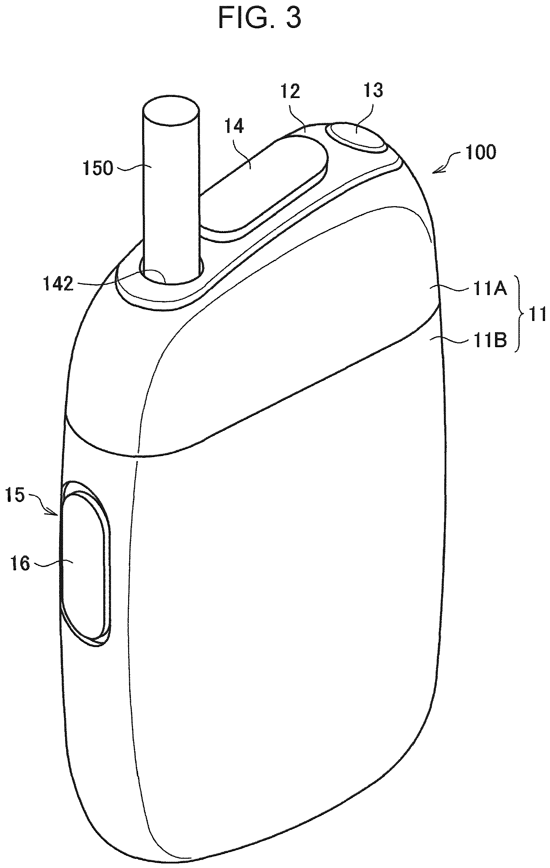

Fig. 2 is an overall perspective view of the inhaler device 100 according to an embodiment. Fig. 3 is an overall perspective view of the inhaler device 100 according to the present embodiment in a state where the stick substrate 150 is held.

As illustrated in Figs. 2 and 3, the inhaler device 100 includes a top housing 11A, a bottom housing 11B, a cover 12, a switch 13, a lid 14, a vent hole 15, and a cap 16. The top housing 11A and the bottom housing 11B are connected to each other to make up an outermost outer housing 11 of the inhaler device 100. The outer housing 11 has such a size as to fit in user's hand. When the user uses the inhaler device 100, the user is able to hold the inhaler device 100 by hand and inhale a flavor.

The top housing 11A has an opening (not illustrated). The cover 12 is coupled to the top housing 11A so as to close the opening. As illustrated in Fig. 3, the cover 12 has an opening 142 through which the stick substrate 150 can be inserted. The lid 14 is configured to open and close the opening 142 of the cover 12.

The switch 13 is used to switch the activation of the inhaler device 100 between an on state and an off state. For example, when the user operates the switch 13 in a state where the stick substrate 150 is inserted in the internal space 141 through the opening 142 as illustrated in Fig. 3, electric power is supplied from the power supply 111 to the heater 121, and the stick substrate 150 can be heated without burning the stick substrate 150. Once the stick substrate 150 is heated, an aerosol is generated from the aerosol source included in the stick substrate 150, and the flavor of the flavor source is taken into the aerosol. The user is able to inhale the aerosol containing a flavor by inhaling a part (a part illustrated in Fig. 3, that is, the inhalation port 152) of the stick substrate 150, protruding from the inhaler device 100.

The vent hole 15 is a vent hole for introducing air into the internal space 141. Air taken into the inhaler device 100 through the vent hole 15 is, for example, introduced into the internal space 141 from the bottom 143 of the container 140. The cap 16 is configured to be attachable to and detachable from the bottom housing 11B. When the cap 16 is attached to the bottom housing 11B, the vent hole 15 is formed between the bottom housing 11B and the cap 16. The cap 16 can have, for example, a through-hole, a cutout, or the like (not illustrated).

### 2. Technical features

### (1) External configuration of inhaler device

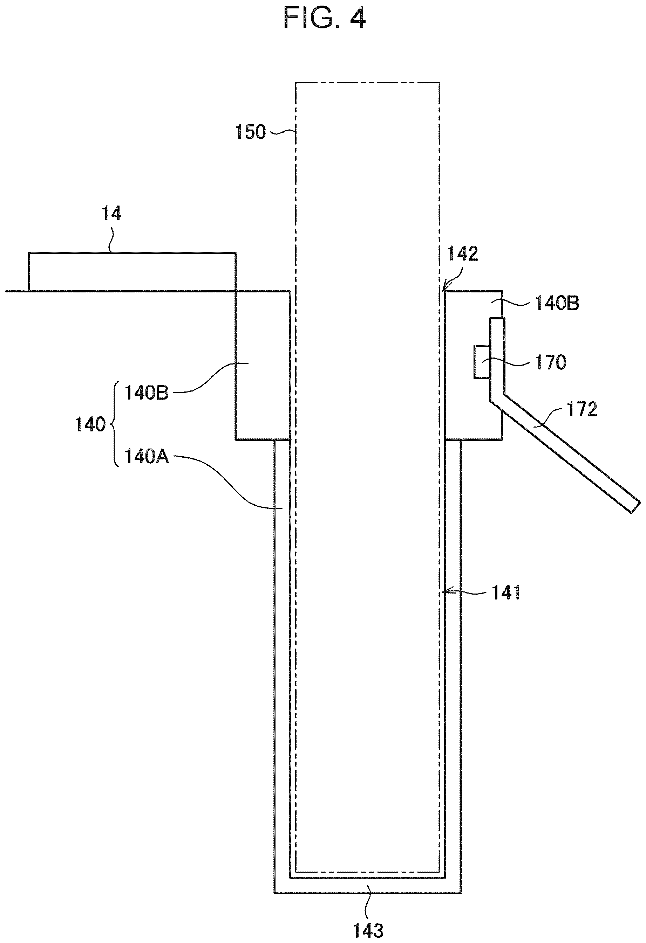

Fig. 4 is a schematic diagram that illustrates the configuration of the inhaler device 100 according to the present embodiment around the container 140 and the configuration of the stick substrate 150. Fig. 4 schematically illustrates a state where the stick substrate 150 is accommodated in the container 140. As illustrated in Fig. 4, the inhaler device 100 includes the lid 14, a stick lower part container 140A, a guide 140B, the internal space 141, the opening 142, the bottom 143, an optical sensor 170, and a circuit board 172.

The stick lower part container 140A is a closed-end tubular body that is a bottom 143-side part of the container 140. The stick lower part container 140A accommodates a bottom 143-side part of the stick substrate 150 inserted in the internal space 141 through the opening 142.

The guide 140B is an open-end tubular body that is an opening 142-side part of the container 140. The guide 140B accommodates a part not accommodated in the stick lower part container 140A, of the part accommodated in the container 140 in the stick substrate 150 inserted in the internal space 141 through the opening 142. Furthermore, the guide 140B functions as a guide for making it easy to insert the stick substrate 150 into the stick lower part container 140A. For example, the guide 140B may have a greater bore diameter than the stick lower part container 140A or may have such a funnel shape that the bore diameter gradually reduces from the opening 142 toward a boundary part with the stick lower part container 140A.

The optical sensor 170 is an example of the first detector in the present embodiment. The optical sensor 170 radiates light to the internal space 141 and detects reflected light received. The optical sensor 170 is, for example, an integrated circuit (IC) equipped with an infrared proximity sensor. In this case, the optical sensor 170 radiates infrared rays to the internal space 141 and detects infrared rays reflected from an object to be detected, such as an object accommodated in the internal space 141 and an inner wall of the container 140.

The optical sensor 170 is disposed at a place where the optical sensor 170 can radiate light to the internal space 141. For example, the optical sensor 170 is disposed at the guide 140B. Specifically, the optical sensor 170 is embedded in the guide 140B. The optical sensor 170 detects light reflected from an object to be detected, such as an object accommodated in the internal space 141 and the inner wall of the guide 140B.

Here, the heater 121 is disposed so as to surround the outer circumference of the stick lower part container 140A. On the other hand, the heater 121 is not disposed around the outer circumference of the guide 140B. The guide 140B may be made of a material having a lower thermal conductivity than the material of the stick lower part container 140A. For this reason, when the optical sensor 170 is disposed at the guide 140B, the optical sensor 170 can perform detection with light without the influence of heating of the stick substrate 150.

The inner wall of the guide 140B may be black. When the inner wall of the guide 140B is black, it is possible to suppress reflection of light radiated from the optical sensor 170.

The circuit board 172 is a board on which the optical sensor 170 is mounted. The circuit board 172 is, for example, flexible printed circuits (FPC) circuit. The circuit board 172 is connected to the controller 116 by, for example, a connector or solder.

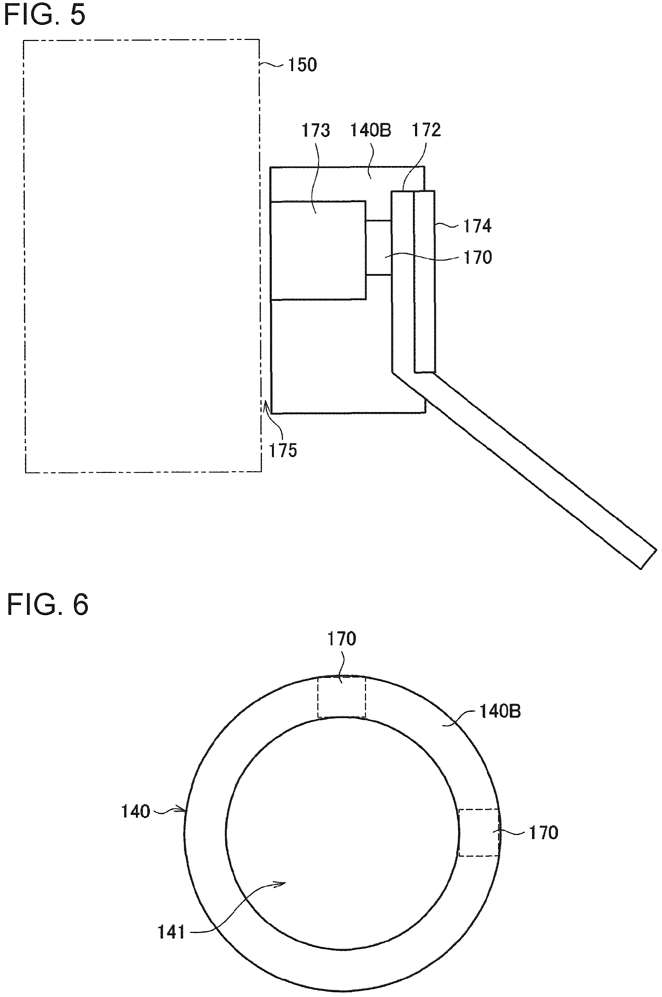

Fig. 5 is a schematic diagram that illustrates the configuration of the inhaler device 100 according to the present embodiment around the container 140 in detail. As illustrated in Fig. 5, the inhaler device 100 further includes a light transmission filter 173 and a reinforcing plate 174. The light transmission filter 173 is a filter that transmits light radiated from the optical sensor 170. The light transmission filter 173 is, for example, an infrared transmission filter in a case where the optical sensor 170 is an infrared proximity sensor. The material of the light transmission filter 173 is not limited. The material of the light transmission filter 173 may be resin or glass or may be formed by applying a light transmission coating to a transparent resin. The light transmission filter 173 may be colored. When the light transmission filter 173 is colored, the optical sensor 170 can be hidden in appearance. With the light transmission filter 173, it is possible to maintain airtightness so that sidestream smoke or the like flowing in from outside the stick does not touch the optical sensor 170.

The reinforcing plate 174 is a component for reinforcing the optical sensor 170 and the circuit board 172. The clearance 175 is a gap provided between the stick substrate 150 and the light transmission filter 173. The clearance 175 may be provided such that the distance between the stick substrate 150 and the light transmission filter 173 ranges from 1 mm to 2 mm.

Fig. 6 is a schematic diagram of the container 140 of the inhaler device 100 according to the present embodiment when viewed from the opening 142 side. As illustrated in Fig. 6, the optical sensor 170 is disposed at the guide 140B. A plurality of the optical sensors 170 may be disposed so as to be capable of detecting the internal space 141 from multiple angles. When the plurality of optical sensors 170 is disposed, it is possible to further accurately determine the state of the container 140. For example, even in a case where soil adheres to part of the container 140 and one of the optical sensors 170 is disposed at a location where the one of the optical sensors 170 cannot detect the soil, another one of the optical sensors 170 can detect the soil.

In a case where a plurality of optical sensors 170 is disposed opposite to each other, there is a possibility that crosstalk occurs when the optical sensor 170 detects light radiated from the opposite optical sensor 170. For this reason, the plurality of optical sensors 170 is preferably disposed not opposite to each other.

### (2) Internal configuration of inhaler device

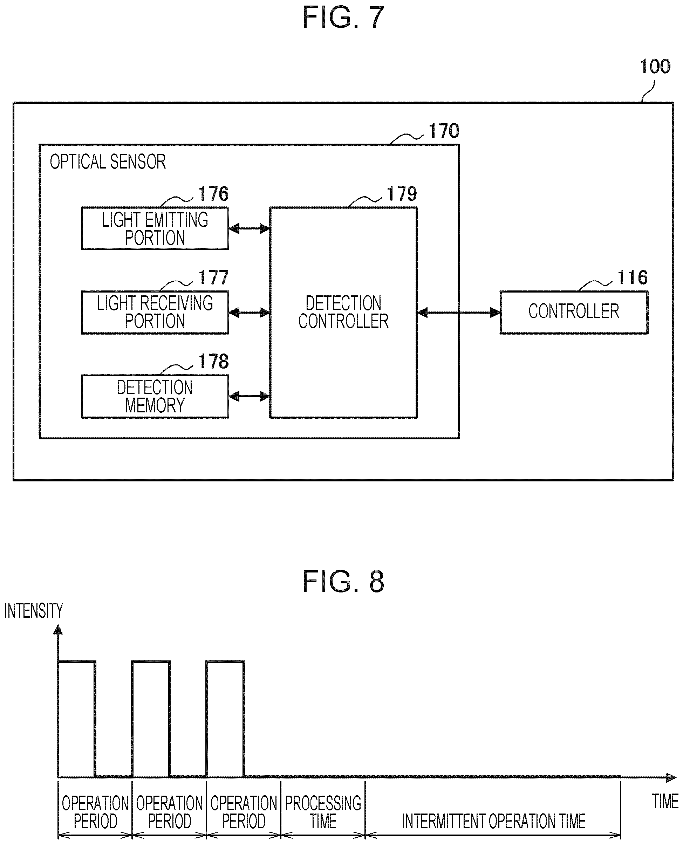

Next, a functional configuration related to detection with the optical sensor 170 of the inhaler device 100 according to the present embodiment will be described with reference to Fig. 7. Fig. 7 is a block diagram that illustrates a functional configuration related to detection with the optical sensor 170 in the inhaler device 100 according to the present embodiment.

As illustrated in Fig. 7, the inhaler device 100 includes the controller 116 and the optical sensor 170. The optical sensor 170 includes a light emitting portion 176, a light receiving portion 177, a detection memory 178, and a detection controller 179. The light emitting portion 176 radiates light to the internal space 141. The light emitting portion 176 is made up of a light-emitting element, such as a laser diode (LD) and a light emitting diode (LED). In the present embodiment, the light emitting portion 176 is an infrared LD and radiates infrared rays. Infrared rays radiated by the light emitting portion 176 may be vertical cavity surface emitting laser (VCSEL). The light receiving portion 177 detects reflected light of light radiated from the light emitting portion 176.

The detection controller 179 controls the operation of each of the structural elements of the optical sensor 170. The detection controller 179 executes a correction process for calculating a corrected detection value corresponding to the received amount of reflected light detected by the light receiving portion 177. Specifically, the detection controller 179 calculates a corrected detection value (corresponding to a first detection value) by correcting a detection value (not corrected) (which corresponds to a second detection value) indicating the received amount of reflected light detected by the light receiving portion 177. The correction process will be described in detail later. The detection controller 179 stores a first detection value in the detection memory 178 (described later).

The detection controller 179 controls an operation period and the length of intermittent operation time. Here, the operation period and the intermittent operation time will be described with reference to Fig. 8. Fig. 8 is a timing chart that illustrates a specific example of detection with the optical sensor 170. The abscissa axis and the ordinate axis of Fig. 8 respectively represent an elapsed time from the start of detection with the optical sensor 170 and the intensity of light radiated from the light emitting portion 176. As illustrated in Fig. 8, the light emitting portion 176 performs pulsed light emission at a constant period. The period is called operation period. The detection controller 179 executes a process related to reflected light detected after the light emitting portion 176 performs pulsed light emission. A time during which the process is executed is called a processing time. After a processing time, a time to when pulsed light emission is performed again is provided. This time is called an intermittent operation time.

Referring back to the description of Fig. 7, the detection controller 179 controls an LD current value that is a current value applied to the light emitting portion 176 to control the intensity of infrared rays radiated from the light emitting portion 176.

When the first detection value is greater than a notified threshold stored in the detection memory 178, the detection controller 179 provides the controller 116 with interrupt notification that is a notification indicating that the first detection value is greater than the notified threshold.

Here, a specific example of detection with the optical sensor 170 will be described with reference to Fig. 8. As illustrated in Fig. 8, the detection controller 179 executes a process related to reflected light detected after the light emitting portion 176 is caused to perform a predetermined number of times of pulsed light emission. The detection controller 179, for example, executes a process of calculating a first detection value by correcting a second detection value. The detection controller 179 executes a process of calculating a distance to an object to be detected, to which light is radiated from the optical sensor 170. During an intermittent operation time after a lapse of a processing time, the detection controller 179 controls the light emitting portion 176 so that radiation of light is not performed. After a lapse of the intermittent operation time, the detection controller 179 controls the light emitting portion 176 such that the light emitting portion 176 radiates light again.

Fig. 8 illustrates an example in which the number of times of pulsed light emission with the light emitting portion 176 is three; however, the number of times of pulsed light emission is not limited. When the number of times of pulsed light emission by the light emitting portion 176 is multiple, the detection controller 179 may execute a process using a detection result, may execute a process using a multiple number of detection results by the light receiving portion 177, or may execute a process using one or some of detection results among a multiple number of detection results with the light receiving portion 177.

Referring back to Fig. 7, the detection memory 178 stores a program that the detection controller 179 executes, various pieces of data, and the like. The detection memory 178 is an example of the memory according to the present embodiment. The detection memory 178 is implemented by, for example, a register. The detection memory 178 stores various setting values, that is, the operation period of pulsed light emission of infrared rays, the intermittent operation time, the notified threshold, the LD current value, and the like, used at the time of control by the detection controller 179. The detection memory 178 stores a first detection value and a correction value. The correction value is a value used to calculate a first detection value from a second detection value.

The controller 116 and the detection controller 179 communicate with each other. The controller 116 and the detection controller 179 communicate with each other by, for example, a serial communication interface, such as inter-integrated circuit (I2C). The controller 116 controls the operations of the structural elements of the optical sensor 170 via the detection controller 179.

The controller 116 controls to switch the mode of the optical sensor 170 to an operation mode to detect reflected light or to a sleep mode to stop detection of reflected light. Specifically, in the sleep mode, the controller 116 controls the light emitting portion 176 such that the light emitting portion 176 stops radiation of light and controls the light receiving portion 177 such that the light receiving portion 177 stops detection of reflected light. In the operation mode, the controller 116 controls the light emitting portion 176 such that the light emitting portion 176 radiates light and controls the light receiving portion 177 such that the light receiving portion 177 detects reflected light. When the controller 116 controls switching of the mode of the optical sensor 170, it is possible to reduce power consumption as compared to a case where detection of reflected light with the optical sensor 170 is constantly performed.

The controller 116 causes the detection memory 178 to store various setting values used at the time of control by the detection controller 179. Here, the detection memory 178 may be made up of a volatile storage medium or may be made up of a non-volatile storage medium. In a case where the detection memory 178 is made up of a non-volatile storage medium, when electric power is supplied again after electric power supplied to the optical sensor 170 is interrupted, various setting values stored in the detection memory 178 are initialized. When various setting values are initialized, the controller 116 may store various setting values before initialization in the detection memory 178 again.

Instead of the sleep mode, the controller 116 may control the mode to a power off mode in which supply of electric power to the optical sensor 170 is stopped. In a case where the detection memory 178 is made up of a volatile storage medium and such control is executed, the controller 116 causes the detection memory 178 to store various setting values before initialization again at the time of switching the mode of the optical sensor 170 from the power off mode to the operation mode. In the sleep mode, the controller 116 may control to maintain supply of electric power to the detection memory 178 of the optical sensor 170. Thus, in a case where the detection memory 178 is made up of a volatile storage medium, it is not necessary to store various setting values before initialization in the detection memory 178 each time the mode is switched from the sleep mode to the operation mode. In the sleep mode, the controller 116 may control to maintain supply of electric power to only the memory of part of the detection memory 178 of the optical sensor 170.

The controller 116 reads various pieces of data, that is, the received amount of reflected light detected with the light receiving portion 177, and the like, stored in the detection memory 178. The controller 116 performs a determination on the state of the container 140 and controls the structural elements of the inhaler device 100, described in detail later, in accordance with the first detection value. The controller 116 receives an interrupt notification from the optical sensor 170.

### (3) Determination on state of container 140 in accordance with detection result with optical sensor 170

Subsequently, a determination related to the state of the container 140 in accordance with the first detection value, performed by the controller 116, will be described in detail. The controller 116 refers to the first detection value stored in the detection memory 178 and performs a determination on the state of the container 140 in accordance with the first detection value.

The controller 116 may determine whether the stick substrate 150 is accommodated in the internal space 141, in accordance with the first detection value. For example, the controller 116 performs a determination as to whether the stick substrate 150 is accommodated in the internal space 141, by comparing the first detection value with a predetermined first threshold. The controller 116 refers to the first threshold stored in advance in the memory 114.

Generally, the outer surface of the stick substrate 150 is white, and the inner wall of the guide 140B is black in the present embodiment. For this reason, of light radiated from the light emitting portion 176, the received amount of light reflected on the outer surface of the stick substrate 150 is estimated to be larger than the received amount of light reflected on the inner wall of the guide 140B. Of course, the color of the outer surface of the stick substrate 150 and the color of the inner wall of the guide 140B are not limited to the above-described example. For example, the color of the outer surface of the stick substrate 150 and the color of the inner wall of the guide 140B may be the same. A received amount at the time of detecting the same-color object to be detected increases as the object to be detected is closer to the optical sensor 170. For this reason, even when the stick substrate 150 and the inner wall of the guide 140B are the same color, of light radiated from the light emitting portion 176, the received amount of light reflected on the outer surface of the stick substrate 150 is larger than the received amount of light reflected on the inner wall of the guide 140B.

For this reason, the first threshold is set to a value less than the first detection value at the time when the stick substrate 150 is accommodated in the internal space 141 and greater than the first detection value at the time when nothing is accommodated in the stick substrate 150. In this case, the controller 116 is capable of appropriately determining whether the stick substrate 150 is accommodated in the internal space 141, by comparing the first detection value with the first threshold. Specifically, the controller 116 determines that the stick substrate 150 is accommodated in the internal space 141 when the first detection value is greater than or equal to the first threshold. The controller 116 determines that the stick substrate 150 is not accommodated in the internal space 141 when the first detection value is less than the first threshold.

The controller 116 may perform a determination on a crud adhering to the container 140 in accordance with the first detection value. The controller 116 may perform a determination on a crud adhering to the container 140 by comparing the first detection value with a predetermined second threshold different from the first threshold. The controller 116 performs comparison with the first detection value by referring to the first threshold and the second threshold stored in advance in the memory 114.

At the time when the stick substrate 150 is accommodated in the internal space 141, in a case where a crud, such as soil and foreign matter, is adhering to the container 140, the reflectance of light radiated from the light emitting portion 176 decreases as compared to a case where no crud, such as soil and foreign matter, is adhering to the container 140. Thus, at the time when the stick substrate 150 is accommodated in the internal space 141, the first detection value in a case where a crud is adhering is less than the first detection value in a case where no crud is adhering. For this reason, the second threshold is set to a value greater than the first threshold. In this case, the controller 116 is capable of appropriately determining the presence or absence of crud in the container 140 by comparing the first detection value with the second threshold at the time when the stick substrate 150 is accommodated in the internal space 141. Specifically, the controller 116 determines that a crud is adhering to the container 140 when the first detection value is greater than or equal to the first threshold and less than the second threshold. The controller 116 determines that no crud is adhering to the container 140 when the first detection value is greater than or equal to the second threshold.

The controller 116 may perform a determination on a degree of crud adhering to the container 140 in accordance with the first detection value. The degree of crud adhering to the container 140 includes the thickness of a sedimented crud, such as soil and foreign matter, an uneven state of sediment, and the like. As the degree of crud increases, the reflectance of light radiated from the light emitting portion 176 decreases, so the first detection value reduces.

In a case where the controller 116 performs a determination on the degree of crud adhering to the container 140, a plurality of second thresholds may be set in a stepwise manner. For example, a second threshold A and a second threshold B that are the second thresholds are set such that (first threshold) < (second threshold A) < (second threshold B). The controller 116 determines that the degree of crud adhering to the container 140 is high when the first detection value is greater than or equal to the first threshold and less than the second threshold A. The controller 116 determines that the degree of crud adhering to the container 140 is low when the first detection value is greater than or equal to the second threshold A and less than the second threshold B. The controller 116 determines that no crud is adhering to the container 140 when the first detection value is greater than or equal to the second threshold B.

Up to here, an example in which the controller 116 performs a determination on a crud adhering to the container 140 in accordance with the second threshold has been described; however, a method of performing a determination on a crud adhering to the container 140 by the controller 116 is not limited to this example. It is estimated that a crud adhering to the container 140 increases with a lapse of time, so it is estimated that the first detection value at the time when the stick substrate 150 is accommodated in the internal space 141 reduces with a lapse of time. The controller 116 may perform a determination on a crud adhering to the container 140 in accordance with a temporal change of the first detection values greater than or equal to the first threshold among the first detection values respectively detected at multiple timings. The controller 116 causes the memory 114 to store a first detection value each time the first detection value is detected by the optical sensor 170. Thus, the controller 116 can perform a determination on a crud adhering to the container 140 in accordance with a temporal change in first detection value.

The controller 116 may calculate a difference between the first threshold and each of the first detection values greater than or equal to the first threshold among the first detection values respectively detected at multiple timings and perform a determination on a crud adhering to the container 140 in accordance with a temporal change of the difference.

The controller 116 may refer to the first detection value stored in the detection memory 178 and perform a determination in accordance with the first detection value only when an interrupt notification is received from the optical sensor 170. The notified threshold may be set to, for example, a value equal to the first threshold. When set in this way, an interrupt notification indicates that the stick substrate 150 is inserted in the internal space 141. For this reason, in a case where an interrupt notification is received from the optical sensor 170, the controller 116 may determine that the stick substrate 150 is inserted. With the above configuration, only when the stick substrate 150 is housed in the internal space 141, the controller 116 performs a determination on the state of the container 140 by referring to the first detection value stored in the detection memory 178, so it is possible to reduce processing load on the controller 116.

### (4) Control over inhaler device 100 in accordance with determination result of state of container 140

Up to here, a determination on the state of the container 140 in accordance with a detection result with the optical sensor 170 according to the present embodiment has been described. Subsequently, control over the structural elements of the inhaler device 100 in accordance with a determination result on the state of the container 140 by the controller 116 will be described.

The controller 116 controls a process of prompting the user to clean the container 140 in accordance with a result of determination on a crud adhering to the container 140. The process of prompting the user to clean the container 140 is, for example, control to notify the user by the notifier 113. The notifier 113 may prompt the user to clean by light emission, sound, or vibration or may display a screen for prompting the user to clean. The controller 116 may control the communicator 115 so that the communicator 115 transmits a notification prompting the user to clean the container 140, to a device, such as a smartphone and a PC, of the user. With this configuration, the user is able to clean the container 140 at appropriate timing.

The controller 116 may control a process of prompting the user to clean the container 140 in a stepwise manner in accordance with a result of determination on a degree of crud adhering to the container 140. For example, when the controller 116 determines that the degree of crud adhering to the container 140 is low, the controller 116 may control the process of prompting the user to clean the container 140; whereas, when the controller 116 determines that the degree of the crud is high, the controller 116 may control the process of further strongly prompting the user to clean the container 140. With the above configuration, the user is able to further clearly recognize the necessity for cleaning the container 140.

The controller 116 may control the timing to prompt the user to clean the container 140 in accordance with a result of determination on a crud adhering to the container 140. For example, the controller 116 may control to prompt the user to clean the container 140 before heating or after heating of the stick substrate 150 with the heater 121 in accordance with a result of determination on a crud adhering to the container 140. More specifically, when the controller 116 determines that the degree of crud adhering to the container 140 is high, the controller 116 may control the process of prompting the user to clean the container 140 before the stick substrate 150 is permitted to be heated with the heater 121. On the other hand, when the controller 116 determines that the degree of crud adhering to the container 140 is low, the controller 116 may control the process of prompting the user to clean the container 140 after the stick substrate 150 is permitted to be heated with the heater 121. There are presumably needs that the user wants to use the inhaler device 100 before cleaning the container 140. With the above configuration, only when it is estimated that appropriate heating cannot be performed by the heater 121 due to the influence of crud in the container 140, it is possible to prompt the user to give priority to cleaning the container 140, so it is possible to come up to needs of the user.

The controller 116 may control heating of the stick substrate 150 with the heater 121 in accordance with a result of determination on a crud adhering to the container 140. For example, the controller 116 may permit or prohibit heating with the heater 121 in accordance with a result of determination on a crud adhering to the container 140.

More specifically, the controller 116 may prohibit heating of the stick substrate 150 with the heater 121 when the controller 116 determines that the degree of crud adhering to the container 140 is high. On the other hand, the controller 116 may permit heating with the heater 121 when the controller 116 determines that the degree of crud adhering to the container 140 is low or when the controller 116 determines that no crud is adhering to the container 140. With the above configuration, it is possible to prohibit heating when the degree of crud adhering to the container 140 is high, so it is possible to reliably make the user clean the container 140.

The controller 116 may cause the memory 114 to store the number of times the first detection value is greater than or equal to the second threshold. The controller 116 updates the number of times stored in the memory 114 when the first detection value greater than or equal to the second threshold is acquired from the optical sensor 170. The controller 116 may transmit the number of times stored in the memory 114 to a device, such as a smartphone and a PC, of the user with the communicator 115. The number of times may be transmitted from the device of the user to a server.

### (5) Switching of mode of optical sensor 170

Next, control over switching of the mode of the optical sensor 170 by the controller 116 will be described in detail. The controller 116 controls to switch the mode of the optical sensor 170 to an operation mode to detect reflected light or to a sleep mode to stop detection of reflected light. The controller 116 may control to switch the mode of the optical sensor 170 in accordance with a detection result obtained by the sensor 112. The sensor 112 is an example of the second detector according to the present embodiment. The controller 116 may control the mode of the optical sensor 170 to a power off mode in which supply of electric power to the optical sensor 170 is stopped, in accordance with a detection result obtained by the sensor 112.

Initially, a case where the sensor 112 detects opening and closing of the opening 142 with the lid 14 will be described. When opening of the opening 142 with the lid 14 is detected, there is a high possibility that the user uses the inhaler device 100. For this reason, once the sensor 112 detects opening of the opening 142 with the lid 14, the controller 116 controls to switch the mode of the optical sensor 170 from the sleep mode to the operation mode. When closing of the opening 142 with the lid 14 is detected, the inhaler device 100 is not used by the user. For this reason, once the sensor 112 detects closing of the opening 142 with the lid 14, the controller 116 controls to switch the mode of the optical sensor 170 from the operation mode to the sleep mode. With the above configuration, detection with the optical sensor 170 is performed only when the user uses the inhaler device 100, so it is possible to efficiently reduce power consumption. The controller 116 may control to switch the mode of the optical sensor 170 from the operation mode to the power off mode once closing of the opening 142 with the lid 14 is detected by the sensor 112.

Next, a case where the sensor 112 detects connection of charging to and disconnection of charging from the power supply 111 by the user will be described. When charging to the power supply 111 is cancelled, there is a high possibility that the user uses the inhaler device 100. For this reason, once the sensor 112 detects cancellation of charging to the power supply 111 by the user, the controller 116 controls to switch the mode of the optical sensor 170 from the sleep mode to the operation mode. When charging to the power supply 111 is connected, there is a high possibility that the inhaler device 100 is not used by the user. For this reason, once the sensor 112 detects connection of charging to the power supply 111 by the user, the controller 116 controls to switch the mode of the optical sensor 170 from the operation mode to the sleep mode. With the above configuration, detection with the optical sensor 170 is performed only when the user uses the inhaler device 100, so it is possible to efficiently reduce power consumption. Once the sensor 112 detects connection of charging to the power supply 111 by the user, the controller 116 may control to switch the mode of the optical sensor 170 from the operation mode to the power off mode.

### (6) Correction of values

Next, correction of values used in the present embodiment by the detection controller 179 and the controller 116 will be described in detail.

### - Correction from second detection value to first detection value

Initially, correction from a second detection value to a first detection value by the detection controller 179 will be described. Reflected light that is detected by the light receiving portion 177 comes under the influence of the individual difference of the optical sensor 170, temperature, vibration, a crud adhering to the container 140, and the like at the time of the detection. Then, the detection controller 179 calculates a first detection value by correcting a second detection value. With the above configuration, it is possible to exclude influence of the individual difference of the optical sensor 170, temperature, vibration, a crud adhering to the container 140, and the like from the second detection value. As a result, the controller 116 is capable of appropriately performing a determination on the state of the container 140.

The detection controller 179 calculates a first detection value by correcting a second detection value. The detection controller 179 calculates a correction value used to calculate a first detection value by correcting a second detection value and causes the detection memory 178 to store the correction value. The detection controller 179 may calculate a correction value by using a detection reference value that is a value to be a reference for a second detection value that is acquired at the time when the stick substrate 150 is not accommodated in the internal space 141. The detection reference value may be set to a value lower than a second detection value detected by the light receiving portion 177 at the timing when the stick substrate 150 is not accommodated in the internal space 141 in a case where there is no decrease in the intensity of light radiated by the light emitting portion 176 with a lapse of usage time. In this case, the detection controller 179 calculates a correction value so that a second detection value acquired when the stick substrate 150 is not accommodated in the internal space 141 is corrected to a value that coincides with the detection reference value. In other words, the detection controller 179 calculates a difference value between the second detection value and the detection reference value as a correction value. The detection controller 179 calculates a first detection value by subtracting the correction value from a second detection value newly output by the light receiving portion 177.

The detection controller 179 calculates a correction value in accordance with a second detection value detected by the light receiving portion 177 at the timing when the stick substrate 150 is not accommodated in the internal space 141. At the timing of detection of the second detection value with the light receiving portion 177, the opening 142 is desirably closed with the lid 14. When the opening 142 is closed, light outside the inhaler device 100 is not received by the light receiving portion 177. When a second detection value is detected by the light receiving portion 177 at the time when the stick substrate 150 is accommodated in the internal space 141 as well, light outside the inhaler device 100 is not received by the light receiving portion 177. For this reason, by using a correction value calculated using a second detection value detected at the timing when the opening 142 is closed, the controller 116 is capable of appropriately performing a determination on the state of the container 140 by excluding the influence due to outside light. However, a difference between a second detection value detected in a case where the opening 142 is closed and a second detection value detected in a case where the opening 142 is open is small as compared with the first threshold and the second threshold. For this reason, at the timing of detection with the light receiving portion 177, the opening 142 may be opened.

A first detection value used to perform a determination on the state of the container 140 by the controller 116 is a value corrected by a correction value that is calculated by the detection controller 179. Here, in order for the controller 116 to further accurately perform a determination on the state of the container 140, conditions of temperature, vibration, a crud adhering to the container 140, and the like desirably coincide with each other between the timing at which the correction value is calculated and the timing at which the state of the container 140 is determined. Thus, the timing to calculate a correction value with the detection controller 179 is desirably close to the timing at which the stick substrate 150 is accommodated in the internal space 141. For this reasons, the controller 116 controls a process of, at the time when a predetermined detection result is obtained by the sensor 112, calculating a correction value with the detection controller 179 and storing the correction value in the detection memory 178.

For example, once the sensor 112 detects opening of the opening 142 with the lid 14, the controller 116 may control calculation of a correction value with the detection controller 179. Immediately after the opening 142 is opened with the lid 14, there is a high possibility that the stick substrate 150 is accommodated in the internal space 141. Therefore, with the above configuration, it is possible to bring the timing to calculate a correction value close to the timing at which the stick substrate 150 is accommodated in the internal space 141, with the result that the controller 116 is capable of accurately performing a determination on the state of the container 140.

Once the sensor 112 detects closing of the opening 142 with the lid 14, the controller 116 may control a process of calculating a correction value with the detection controller 179 and storing the correction value in the detection memory 178. At the time when the controller 116 switches the mode of the optical sensor 170 to the operation mode after the storage, the controller 116 controls a process of correcting a second detection value by using the correction value stored in the detection memory 178. When the sensor 112 detects closing of the opening 142 with the lid 14, there is a high possibility that it is the timing that the user ends inhalation. For this reason, with the above process, the detection controller 179 is capable of correcting the influence of adherent crud due to inhalation before closing of the opening 142 with the lid 14. Here, at the time when the user opens the opening 142 with the lid 14, a correction value is already calculated by the detection controller 179. For this reason, at the time when the user opens the opening 142 with the lid 14, the controller 116 is capable of performing a determination on the state of the container 140 without controlling execution of the process of calculating a correction value. In other words, with the above configuration, in comparison with a case where the sensor 112 detects opening of the opening 142 with the lid 14 and then the controller 116 calculates a correction value, a waiting time of the user is reduced.

When the sensor 112 detects closing of the opening 142 with the lid 14, the controller 116 may execute the process of causing the detection memory 178 to store a calculated correction value and then switch the mode of the optical sensor 170 from the operation mode to the sleep mode.

Once the sensor 112 detects cancellation of charging to the power supply 111, the controller 116 may control calculation of a correction value with the detection controller 179. Charging to the power supply 111 may be charging from a commercial power supply or may be charging from a charging device capable of housing the inhaler device 100, such as a portable charging and re-filling case (PCC). Immediately after cancellation of charging is detected, the stick substrate 150 is highly likely to be accommodated in the internal space 141. Therefore, with the above configuration, it is possible to bring the timing to calculate a correction value close to the timing at which the stick substrate 150 is accommodated in the internal space 141, with the result that the controller 116 is capable of accurately performing a determination on the state of the container 140.

Once the sensor 112 detects connection of charging to the power supply 111, the controller 116 may control a process of calculating a correction value with the detection controller 179 and storing the correction value in the detection memory 178. At the time when the controller 116 switches the mode of the optical sensor 170 to the operation mode after the storage, the controller 116 controls a process of correcting a second detection value by using the correction value stored in the detection memory 178. When the sensor 112 detects connection of charging to the power supply 111, there is a high possibility that it is the timing that the user ends inhalation. For this reason, with the above process, the detection controller 179 is capable of correcting a second detection value having varied under the influence of adherent crud due to inhalation before closing of the opening 142 with the lid 14. By processing in this way, at the time when cancellation of charging to the power supply 111 is detected, a correction value is already calculated by the detection controller 179. For this reason, at the time when cancellation of charging to the power supply 111 is detected, the controller 116 is capable of performing a determination on the state of the container 140 without controlling execution of the process of calculating a correction value. In other words, with the above configuration, in comparison with a case where the sensor 112 detects cancellation of charging to the power supply 111 and then the controller 116 calculates a correction value, a waiting time of the user is reduced.

When the sensor 112 detects connection of charging to the power supply 111, the controller 116 may execute the process of causing the detection memory 178 to store a calculated correction value and then switch the mode of the optical sensor 170 from the operation mode to the sleep mode.

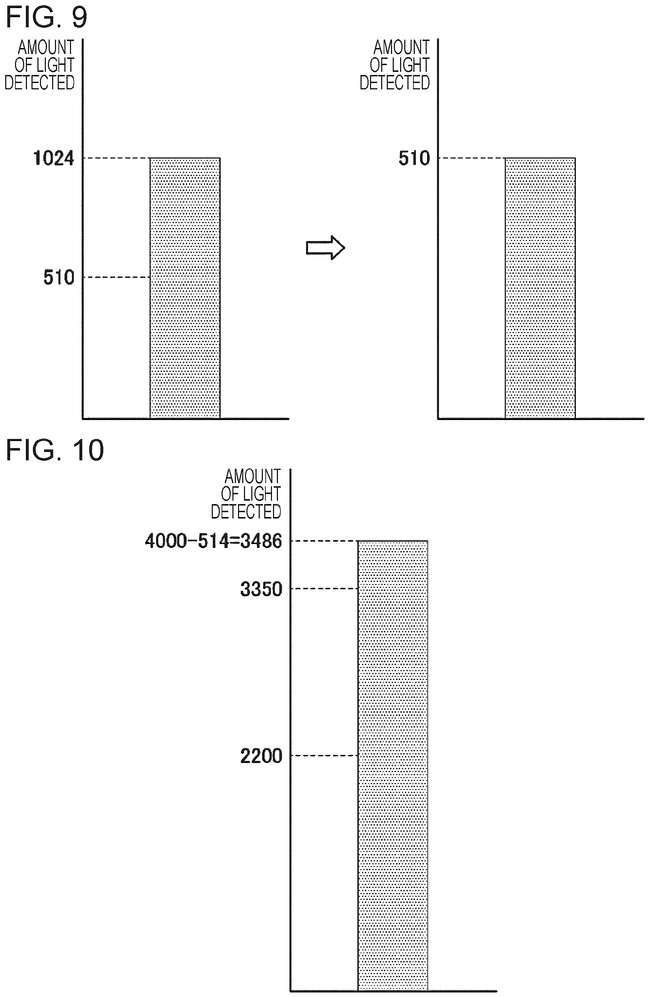

Here, a specific example of correction of a second detection value by the detection controller 179 will be described with reference to Figs. 9 to 12. Fig. 9 is a view that illustrates a specific example of correction of a second detection value with the detection controller 179. Here, a specific example in a case where the detection reference value is "510" will be described. A detected amount of light indicates that the amount of light received increases as the value increases. Here, it is assumed that the first threshold used to determine whether the stick substrate 150 is accommodated in the internal space 141 is "2200" and the second threshold used to determine the presence or absence of crud in the container 140 is "3350".

The left-hand view of Fig. 9 is a view illustrating an example of a second detection value acquired when the stick substrate 150 is not accommodated in the internal space 141. The right-hand view of Fig. 9 is a view illustrating an example obtained by correcting a second detection value acquired when the stick substrate 150 is not accommodated in the internal space 141 into a detection reference value. The detection controller 179 calculates a correction value so that a second detection value acquired when the stick substrate 150 is not accommodated in the internal space 141 is corrected to 510 that coincides with the detection reference value. As illustrated in Fig. 9, when a second detection value acquired when the stick substrate 150 is not accommodated in the internal space 141 is "1024", the detection controller 179 calculates a difference value "514" between "1024" that is the second detection value and "510" that is the detection reference value as a correction value. The detection controller 179 causes the detection memory 178 to store the calculated correction value.

A specific example of determination as to the state of the container 140 with the controller 116 in a case where, after control of the process of storing a correction value with the detection controller 179, described with reference to Fig. 9, "4000" is newly output as a second detection value by the light receiving portion 177 will be described with reference to Fig. 10.

Fig. 10 is a view that illustrates a specific example of determination on the state of the container 140 using a first detection value by the controller 116. Here, the detection controller 179 calculates "3486" as a first detection value by subtracting "514" that is a correction value from "4000" that is a second detection value. The controller 116 reads the first detection value from the detection memory 178 and compares the first detection value with the first threshold and the second threshold. Here, the first detection value is greater than or equal to the first threshold and the second threshold. For this reason, the controller 116 determines that the stick substrate 150 is accommodated in the internal space 141 and no crud is adhering in the container 140.

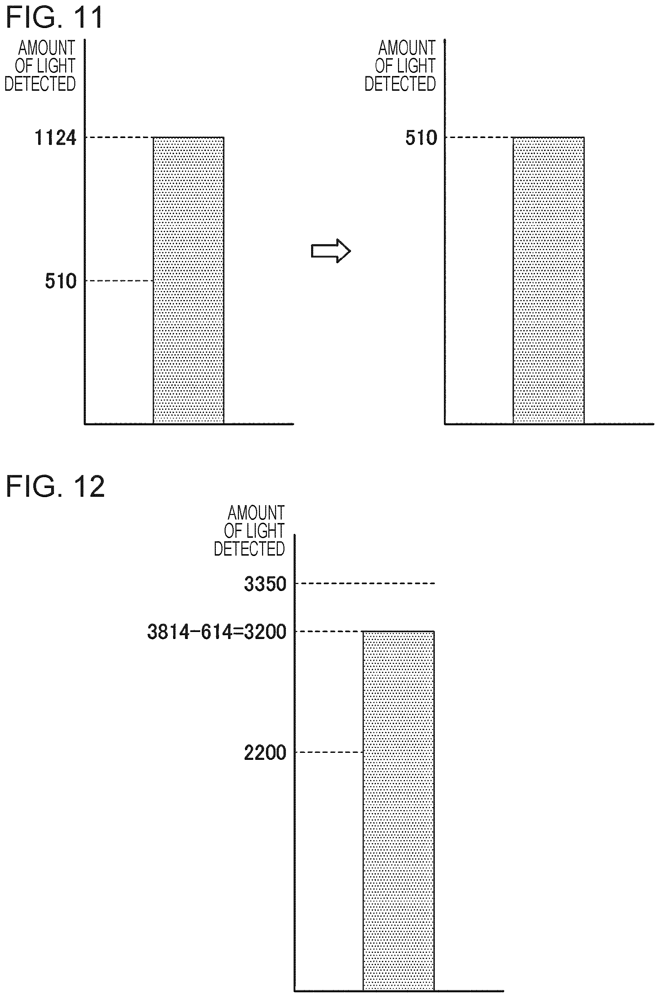

Fig. 11 is a view that illustrates a specific example of correction of a second detection value with the detection controller 179. Here, a specific example in a case where the detection reference value is "510" will be described. Here, it is assumed that the first threshold used to determine whether the stick substrate 150 is accommodated in the internal space 141 is "2200" and the second threshold used to determine the presence or absence of crud in the container 140 is "3350".

The left-hand view of Fig. 11 is a view illustrating an example of a second detection value acquired when the stick substrate 150 is not accommodated in the internal space 141. The right-hand view of Fig. 11 is a view illustrating an example obtained by correcting a second detection value acquired when the stick substrate 150 is not accommodated in the internal space 141 into a detection reference value. As illustrated in Fig. 11, the second detection value acquired when the stick substrate 150 is not accommodated in the internal space 141 is "1124" greater than "1024" that is the second detection value illustrated in the left-hand view of Fig. 9. When soil adheres to around the optical sensor 170 disposed at the container 140, light reflects on the adherent soil, so the amount of light received increases as compared to a case with no soil, and the second detection value increases. The detection controller 179 calculates a correction value so that a second detection value acquired when the stick substrate 150 is not accommodated in the internal space 141 is corrected to "510" that coincides with the detection reference value. As illustrated in Fig. 11, when a second detection value acquired when the stick substrate 150 is not accommodated in the internal space 141 is "1124", the detection controller 179 calculates a difference value "614" between "1124" that is the second detection value and "510" that is the detection reference value as a correction value. The detection controller 179 causes the detection memory 178 to store the calculated correction value.

A specific example of determination as to the state of the container 140 with the controller 116 in a case where, after control of the process of storing a correction value with the detection controller 179, described with reference to Fig. 11, "3814" is newly output as a second detection value by the light receiving portion 177 will be described with reference to Fig. 12.

Fig. 12 is a view that illustrates a specific example of determination on the state of the container 140 using a first detection value by the controller 116. Here, the detection controller 179 calculates "3200" as a first detection value by subtracting "614" that is a correction value from "3814" that is a second detection value. The controller 116 reads the first detection value from the detection memory 178 and compares the first detection value with the first threshold and the second threshold. Here, the first detection value is greater than or equal to the first threshold and less than the second threshold. For this reason, the controller 116 determines that the stick substrate 150 is accommodated in the internal space 141 and a crud is adhering to the container 140.

The inhaler device 100 may perform a determination on a crud without performing correction of a detection value. However, when correction of a detection value is performed, it is possible to improve the accuracy of determination as to a crud as compared to a case where correction of a detection value is not performed.

Here, there is a case where a detection value is corrected, that is, a first detection value is calculated from a second detection value, in a state where a crud is adhering. In this case as well, it is possible to improve the accuracy of determination as to a crud in a state where the stick substrate 150 is inserted. Hereinafter, this point will be described in detail.

The amount of light received at the time of calculating a correction value tends to increase as a crud increases. This is because light radiated from the optical sensor 170 is reflected by a crud adhering to a location closer to the optical sensor 170 than the inner wall of the container 140. In other words, when a second detection value is corrected in a state where a crud is adhering, a correction value increases as compared to a case where a second detection value is corrected in a state where no crud is adhering. As a result, a first detection value in a state where the stick substrate 150 is inserted decreases under the influence of an increase in correction value. On the other hand, the amount of light in a case where the stick substrate 150 is inserted tends to reduce as a crud increases. This is because light radiated to the stick substrate 150 or light reflected from the stick substrate 150 is blocked by a crud. As a result, a first detection value in a state where the stick substrate 150 is inserted decreases under the influence that a crud blocks light. In this way, when a second detection value is corrected in a state where a crud is adhering, a first detection value in a state where the stick substrate 150 is inserted significantly decreases under the influence of an increase in correction value and blocking of light by a crud. As a result, it is possible to reliably determine that a crud is adhering by the second threshold set to a value greater than the first threshold.

### - Correction of threshold used to determine state of container 140

Next, correction of the first threshold and the second threshold used to determine the state of the container 140 by the controller 116 will be described. The intensity of light radiated from the light emitting portion 176 decreases with a lapse of usage time. With a decrease in the intensity of light radiated from the light emitting portion 176, the intensity of reflected light detected with the light receiving portion 177 decreases. For this reason, if the first threshold and the second threshold are used without considering a decrease in the intensity of light radiated from the light emitting portion 176, the controller 116 possibly cannot appropriately determine the state of the container 140. For example, even when the stick substrate 150 is accommodated in the container 140, there is a possibility that a first detection value is less than the first threshold and the controller 116 determines that the stick substrate 150 is not accommodated in the container 140.

The controller 116 corrects the first threshold and the second threshold. With the above configuration, it is possible to prevent the controller 116 from erroneously determining the state of the container 140 due to a decrease in the intensity of light radiated from the light emitting portion 176. For example, the controller 116 corrects the first threshold and the second threshold in accordance with a temporal change in correction value. Specifically, the controller 116 calculates the ratio of a second correction value that is a newly calculated correction value to a difference value between the second correction value and a first correction value that is a correction value calculated in the past and performs correction by multiplying the calculated ratio by the first threshold and the second threshold. The controller 116 can correct the first threshold and the second threshold if the first correction value is stored in the memory 114 in advance.

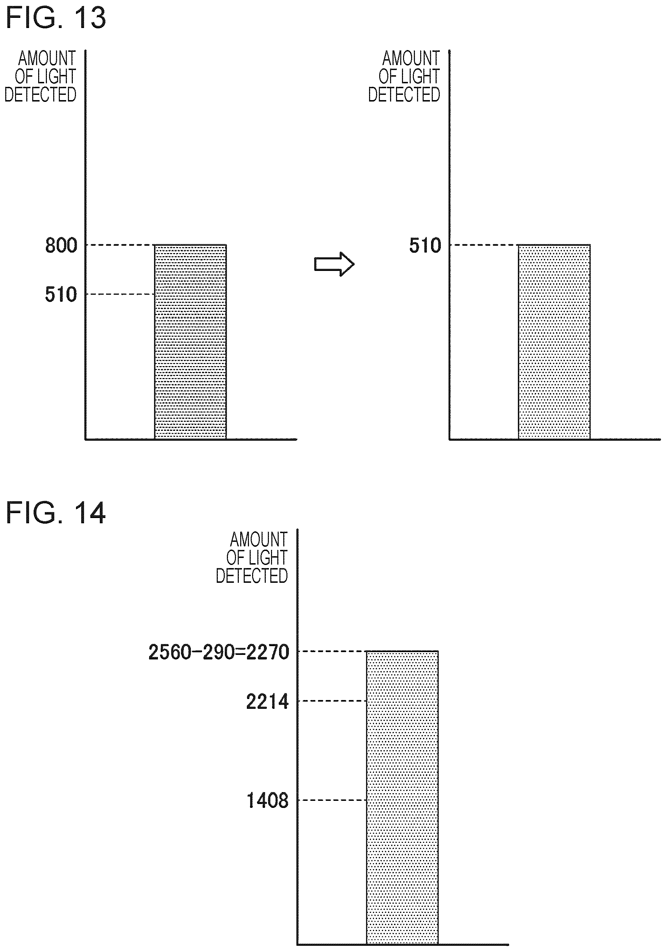

Here, a specific example of correction of the first threshold and the second threshold by the detection controller 116 will be described with reference to Figs. 13 to 16. Fig. 13 is a view that illustrates a specific example of correction of the first threshold and the second threshold with the detection controller 179. Here, a specific example in a case where the detection reference value is "510" will be described. Here, it is assumed that the first threshold before correction used to determine whether the stick substrate 150 is accommodated in the internal space 141 is "2200" and the second threshold before correction used to determine the presence or absence of crud in the container 140 is "3350". Here, it is assumed that the first correction value that is a correction value calculated in the past is "450".

The left-hand view of Fig. 13 is a view illustrating an example of a second detection value acquired when the stick substrate 150 is not accommodated in the internal space 141. The right-hand view of Fig. 13 is a view illustrating an example obtained by correcting a second detection value acquired when the stick substrate 150 is not accommodated in the internal space 141 into a detection reference value. The detection controller 179 calculates a correction value so that a second detection value acquired when the stick substrate 150 is not accommodated in the internal space 141 is corrected to 510 that coincides with the detection reference value. As illustrated in Fig. 13, when a second detection value acquired when the stick substrate 150 is not accommodated in the internal space 141 is "800", the detection controller 179 calculates a difference value "290" between "800" that is the second detection value and "510" that is the detection reference value as a second correction value.

The controller 116 reads the second correction value from the detection memory 178 and calculates the ratio of the second correction value to the first correction value stored in the memory 114. The controller 116 corrects the threshold by multiplying "0.64" that is the calculated ratio by the first threshold and the second threshold. In other words, the controller 116 corrects the first threshold to "1408", corrects the second threshold to "2214", and updates the first threshold and the second threshold, stored in the memory 114.

A specific example of determination as to the state of the container 140 with the controller 116 in a case where, after the controller 116 updates the first threshold and the second threshold, described with reference to Fig. 13, "2560" is newly output as a second detection value by the light receiving portion 177 will be described with reference to Fig. 14.

Fig. 14 is a view that illustrates a specific example of determination on the state of the container 140 using a first detection value by the controller 116. Here, the detection controller 179 calculates "2270" as a first detection value by subtracting "290" that is a second correction value from "2560" that is a second detection value. The controller 116 reads the first detection value from the detection memory 178 and compares the first detection value with the corrected first threshold and the corrected second threshold. Here, the first detection value is greater than or equal to the corrected first threshold and the corrected second threshold. For this reason, the controller 116 determines that the stick substrate 150 is accommodated in the internal space 141 and no crud is adhering to the container 140.

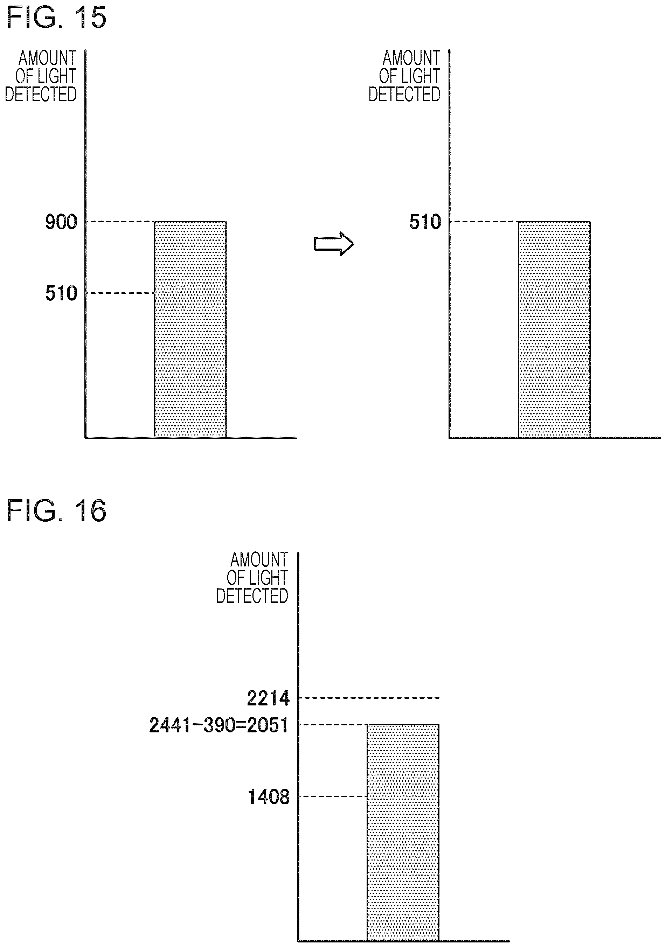

Fig. 15 is a view that illustrates a specific example of correction of a second detection value after correction of the first threshold and the second threshold, illustrated with reference to Fig. 13. Here, a specific example in a case where the detection reference value is "510" as in the case of the specific example described with reference to Fig. 13 will be described. Here, as described with reference to Fig. 13, the corrected first threshold is "1408", and the corrected second threshold is "2214".

The left-hand view of Fig. 15 is a view illustrating an example of a second detection value acquired when the stick substrate 150 is not accommodated in the internal space 141. The right-hand view of Fig. 15 is a view illustrating an example obtained by correcting a second detection value acquired when the stick substrate 150 is not accommodated in the internal space 141 into a detection reference value. The detection controller 179 calculates a correction value so that a second detection value acquired when the stick substrate 150 is not accommodated in the internal space 141 is corrected to "510" that coincides with the detection reference value. As illustrated in Fig. 15, when a second detection value acquired when the stick substrate 150 is not accommodated in the internal space 141 is "900", the detection controller 179 calculates a difference value "390" between "900" that is the second detection value and "510" that is the detection reference value as a correction value. The detection controller 179 causes the detection memory 178 to store the calculated correction value.

A specific example of determination as to the state of the container 140 with the controller 116 in a case where, after control of the process of storing a correction value with the detection controller 179, described with reference to Fig. 15, "2441" is newly output as a second detection value by the light receiving portion 177 will be described with reference to Fig. 16.

Fig. 16 is a view that illustrates a specific example of determination on the state of the container 140 using a first detection value by the controller 116. Here, the detection controller 179 calculates "2051" as a first detection value by subtracting "390" that is a correction value from "2441" that is a second detection value. The controller 116 reads the first detection value from the detection memory 178 and compares the first detection value with the corrected first threshold and the corrected second threshold. Here, the first detection value is greater than or equal to the corrected first threshold and less than the corrected second threshold. For this reason, the controller 116 determines that the stick substrate 150 is accommodated in the internal space 141 and a crud is adhering to the container 140.

Correction of the first threshold and the second threshold by the controller 116 is preferably performed at the timing when the intensity of light radiated from the light emitting portion 176 has decreased through usage. For example, at the time when the number of times a correction value is calculated reaches a predetermined number of times, the controller 116 may correct the first threshold and the second threshold. In this case, the controller 116 causes the memory 114 to store the number of times a correction value is calculated and updates the number of times each time a correction value is calculated.

The controller 116 may correct the first threshold and the second threshold at the time when a time during which the mode of the optical sensor 170 is the operation mode reaches a predetermined time. In this case, the controller 116 causes the memory 114 to store the time during which the mode of the optical sensor 170 is the operation mode.

A correction value reduces with a decrease in the intensity of light radiated from the light emitting portion 176. For this reason, at the time when the correction value becomes less than a predetermined correction reference value, the controller 116 may correct the first threshold and the second threshold. Here, when a correction value is calculated, the degree of crud adhering to the container 140 is not clear. For this reason, the controller 116 may cause the memory 114 to store a correction value each time the correction value is calculated, select a predetermined number of correction values from among the stored correction values in reverse chronological order, and calculate an average of the plurality of selected correction values. Then, at the time when the correction value becomes less than a predetermined correction reference value, the controller 116 may correct the first threshold and the second threshold. When the number of times a correction value becomes less than the predetermined correction reference value exceeds a certain number of times, the controller 116 may correct the first threshold and the second threshold.

A plurality of conditions related to the timing at which the controller 116 corrects the first threshold and the second threshold has been described. Alternatively, a condition related to the timing at which the controller 116 corrects the first threshold and the second threshold may be a combination of conditions. For example, at the time when the number of times a correction value is calculated reaches a predetermined number of times and the correction value becomes less than a predetermined correction reference value, the controller 116 may correct the first threshold and the second threshold.

Up to here, an example in which a second correction value used to correct the first threshold and the second threshold is calculated from a single second detection value has been described. However, the degree of crud adhering to the container 140 is not clear at the time when a correction value is calculated, so a second correction value used to correct the first threshold and the second threshold is preferably an average of multiple correction values stored in the memory 114.

Up to here, an example of a case where a second detection value is greater than a detection reference value has been described. Alternatively, a second detection value may be less than a detection reference value due to the influence of temperature, vibration, or the like. In other words, a difference value between a second detection value and a detection reference value may be a negative value.

### 3. Operation of inhaler device

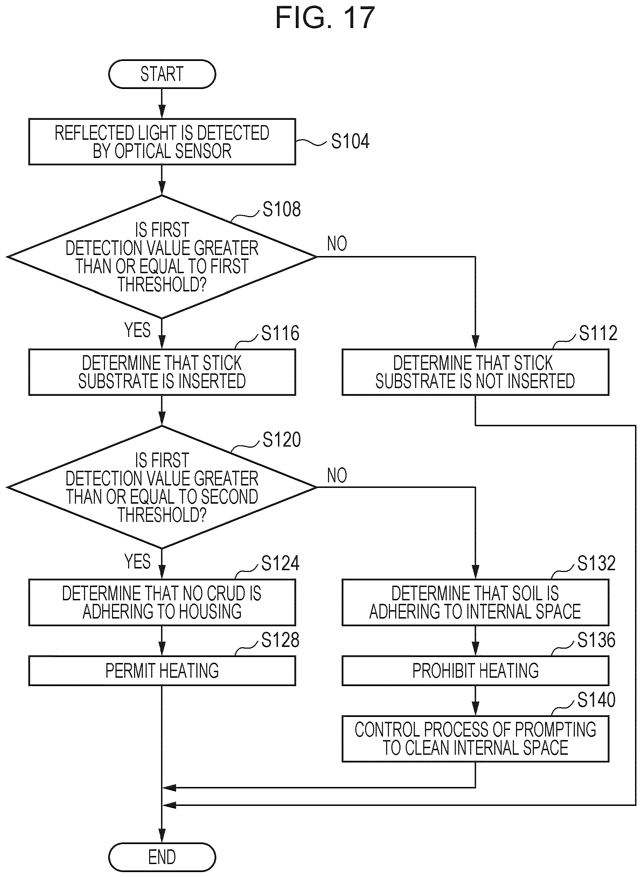

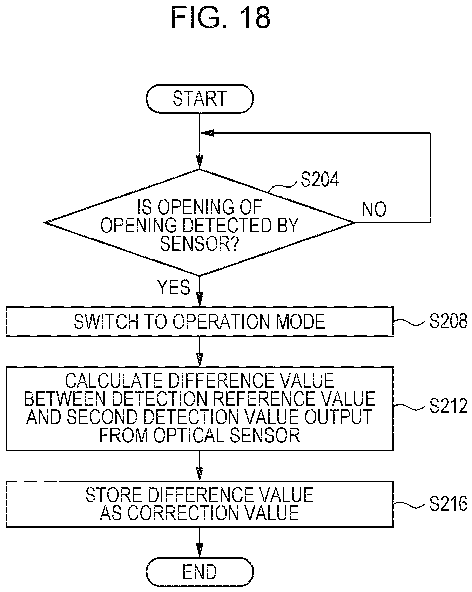

Next, an operation example of the inhaler device 100 according to the present embodiment will be described with reference to Figs. 17 and 18.

### 3.1. Operation control of inhaler device 100 in accordance with detection result with optical sensor 170

Fig. 17 is a flowchart that illustrates operation control of the inhaler device 100 in accordance with a detection result with the optical sensor 170. In this operation example, it is assumed that the first threshold is a threshold used to determine whether the stick substrate 150 is accommodated in the internal space 141 and the second threshold is a threshold used to determine the presence or absence of crud in the container 140.

Initially, once reflected light is detected by the light receiving portion 177 of the optical sensor 170 (S104), the controller 116 refers to a first detection value corresponding to the received amount of reflected light, stored in the detection memory 178. When the first detection value is less than the first threshold (No in S108), the controller 116 determines that the stick substrate 150 is not accommodated in the container 140 (S112), and ends the process. On the other hand, when the first detection value is greater than or equal to the first threshold (Yes in S108), the controller 116 determines that the stick substrate 150 is accommodated in the container 140 (S116).

Subsequently, the controller 116 determines whether the first detection value is greater than or equal to the second threshold (S120). When the first detection value is greater than or equal to the second threshold (Yes in S120), the controller 116 determines that no crud is adhering to the container 140 (S124). Then, the controller 116 permits heating of the stick substrate 150 with the heater 121 (S128).

On the other hand, when the first detection value is less than the second threshold (No in S120), the controller 116 determines that a crud is adhering to the container 140 (S132). Then, the controller 116 prohibits heating of the stick substrate 150 with the heater 121 (S136) and controls a process of prompting the user to clean the container 140 (S140).

### 3.2. Correction of second detection value

Fig. 18 is a flowchart that illustrates the flow of correction of a second detection value with the detection controller 179. In this operation example, a case where the mode of the optical sensor 170 is the sleep mode will be described. Initially, the controller 116 determines whether opening of the opening 142 is detected with the sensor 112. Once the sensor 112 detects opening of the opening 142 with the lid 14 (Yes in S204), the controller 116 controls to switch the mode of the optical sensor 170 to the operation mode (S208).

Subsequently, the controller 116 controls a process with the detection controller 179. The detection controller 179 calculates a difference value between a detection reference value and a second detection value output from the light receiving portion 177 of the optical sensor 170 (S212) and causes the detection memory 178 to store the difference value as a correction value (S216).

On the other hand, when the sensor 112 does not detect opening of the opening 142 with the lid 14 (No in S204), the optical sensor 170 continues the sleep mode until the sensor 112 detects opening of the opening 142 with the lid 14.

### 4. Supplement

The preferred embodiment of the present invention has been described in detail with reference to the attached drawings, however, the present invention is not limited to those examples. It is obvious that persons having ordinary skill in the art in the field of technology to which the present invention belongs can conceive of various modifications or alterations within the scope of the technical idea recited in the claims, and these can also be naturally interpreted as belonging to the technical scope of the present invention.

For example, up to here, an example in which the first threshold and the second threshold are set regardless of the type of the stick substrate 150 has been described. Alternatively, the first threshold and the second threshold that vary depending on the type of the stick substrate 150 may be set. In this case, the controller 116 determines the type of the stick substrate 150 housed in the container 140. Then, the controller 116 performs a determination on the state of the container 140 using the first threshold and the second threshold suitable for the stick substrate 150 of the determined type.

Up to here, an example in which, at the time when the sensor 112 detects opening or closing of the opening 142 with the lid 14 or connection or cancellation of charging to the power supply 111, the controller 116 controls to switch the mode of the optical sensor 170 or to correct the second detection value has been described. However, detection with the sensor 112 is not limited to this example.

For example, the sensor 112 may be a motion sensor and may detect lifting of the inhaler device 100 by the user. In this case, once the sensor 112 detects lifting of the inhaler device 100 by the user, the controller 116 may switch the mode of the optical sensor 170 to the operation mode. Once the sensor 112 detects lifting of the inhaler device 100 by the user, the controller 116 may control correction of the second detection value. In a case where the sensor 112 is a button, the controller 116 may control various processes in response to detection of depression of the button by the user.

The timing to control to switch the mode of the optical sensor 170 or to correct the second detection value is not limited to the timing at which a detection result is obtained by the sensor 112. For example, the controller 116 may execute various processes in response to the timing when the heater 121 completes heating. The controller 116 may control various processes at the time when a plurality of conditions described up to here is satisfied. For example, the controller 116 may switch the mode of the optical sensor 170 to the operation mode at the time when the sensor 112 detects that the opening 142 is opened with the lid 14 and cancellation of charging to the power supply 111 is detected.

A series of pieces of processing, executed by the devices, described in the specification, may be implemented by any one of software, hardware, and a combination of software and hardware. Programs that are components of software are prestored in, for example, storage media (non-transitory media) provided inside or outside the devices. The programs are, for example, loaded onto a RAM when a computer runs the programs and are run on a processor, such as a CPU. Examples of the storage media include a magnetic disk, an optical disk, a magneto-optical disk, and a flash memory. The computer programs may be distributed via, for example, a network, without using storage media.

Pieces of processing described by using the flowchart and the sequence diagram in the specification may be not necessarily executed in order as illustrated. Some processing steps may be executed in parallel. An additional processing step may be adopted, and one or some of the processing steps may be omitted.

The following configurations also belong to the technical scope of the present invention.
(1) An inhaler device including:
   a container capable of accommodating a stick substrate in an internal space;
   a first detector that radiates light to the internal space and detects reflected light received;
   a second detector that detects an action of a user to the inhaler device,
   a controller that performs a determination on a state of the container in accordance with a first detection value corresponding to an intensity of reflected light detected by the first detector; and
   a memory that stores a correction value used to calculate the first detection value by correcting a second detection value indicating an intensity of reflected light detected by the first detector, wherein
   the controller controls a process of, when a predetermined detection result is obtained by the second detector, calculating the correction value and storing the correction value in the memory.
(2) The inhaler device according to (1), wherein
   the correction value is a difference value between a predetermined detection reference value and the second detection value output from the first detector,
   the first detection value is a value obtained by subtracting the correction value from the second detection value, and
   the controller controls a process of, when a predetermined detection result is obtained by the second detector, calculating the first detection value.
(3) The inhaler device according to (1) or (2), wherein the controller controls to switch a mode of the first detector to an operation mode to detect the reflected light or a sleep mode to stop detection of the reflected light, in accordance with a detection result obtained by the second detector.
(4) The inhaler device according to (3), wherein
   the inhaler device further includes a lid capable of opening and closing an opening that communicates with the internal space of the container, and
   the second detector detects opening and closing of the opening with the lid.
(5) The inhaler device according to (4), wherein the controller controls to switch a mode of the first detector to the operation mode when opening of the opening with the lid is detected by the second detector and controls to switch the mode of the first detector to the sleep mode when closing of the opening with the lid is detected by the second detector.
(6) The inhaler device according to (4) or (5), wherein the controller controls a process of calculating the correction value when opening of the opening with the lid is detected by the second detector.
(7) The inhaler device according to (4) or (5), wherein the controller controls a process of calculating the correction value and storing the calculated correction value in the memory when closing of the opening with the lid is detected by the second detector and controls a process of correcting the second detection value by using the correction value stored in the memory when the mode of the first detector is switched to the operation mode after the storage.
(8) The inhaler device according to (3), wherein
   the inhaler device further includes a power supply that stores electric power, and
   the second detector detects connection and cancellation of charging to the power supply by the user.
(9) The inhaler device according to (8), wherein the controller controls to switch the mode of the first detector to the operation mode when cancellation of charging to the power supply is detected by the second detector and to switch the mode of the first detector to the sleep mode when connection of charging to the power supply is detected by the second detector.
(10) The inhaler device according to (8) or (9), wherein the controller calculates the correction value when cancellation of charging to the power supply is detected by the second detector.
(11) The inhaler device according to (8) or (9), wherein the controller controls a process of calculating the correction value and storing the calculated correction value in the memory when connection of charging to the power supply is detected by the second detector and controls a process of correcting the second detection value by using the correction value stored in the memory when the mode of the first detector is switched to the operation mode after the storage.
(12) The inhaler device according to any one of (1) to (11), wherein the controller determines whether the stick substrate is accommodated in the internal space in accordance with the first detection value.
(13) The inhaler device according to any one of (1) to (11), wherein the controller performs a determination on a crud adhering to the container in accordance with the first detection value.
(14) The inhaler device according to any one of (1) to (13), wherein the controller performs a determination on a state of the container in accordance with the first detection value and a predetermined threshold and corrects the threshold in accordance with a temporal change in the correction value.
(15) A control method for an inhaler device, the control method being executed by a computer, the inhaler device including
   a container capable of accommodating a stick substrate in an internal space,
   a first detector that radiates light to the internal space and detects reflected light received, and
   a second detector that detects an action of a user to the inhaler device, and
   a memory that stores a correction value used to calculate a first detection value corresponding to an intensity of reflected light detected by the first detector by correcting a second detection value indicating an intensity of reflected light detected by the first detector, the control method including:
      performing a determination on a state of the container in accordance with the first detection value; and
      controlling a process of, when a predetermined detection result is obtained by the second detector, calculating the correction value and storing the correction value in the memory.

### Reference Signs List

- 100: inhaler device
- 14: lid
- 111: power supply
- 112: sensor
- 113: notifier
- 114: memory
- 115: communicator
- 116: controller
- 121: heater
- 140: container
- 140A: stick lower part container
- 140B: guide
- 141: internal space
- 142: opening
- 150: stick substrate
- 170: optical sensor
- 172: circuit board
- 173: light transmission filter
- 174: reinforcing plate
- 175: clearance
- 176: light emitting portion
- 177: light receiving portion
- 178: detection memory
- 179: detection controller

## Claims

1. An inhaler device comprising:
a container capable of accommodating a stick substrate in an internal space;
a first detector that radiates light to the internal space and detects reflected light received; and
a second detector that detects an action of a user to the inhaler device;
a controller that performs a determination on a state of the container in accordance with a first detection value corresponding to an intensity of reflected light detected by the first detector; and
a memory that stores a correction value used to calculate the first detection value by correcting a second detection value indicating an intensity of reflected light detected by the first detector, wherein
the controller controls a process of, when a predetermined detection result is obtained by the second detector, calculating the correction value and storing the correction value in the memory.

2. The inhaler device according to claim 1, wherein
the correction value is a difference value between a predetermined detection reference value and the second detection value output from the first detector,
the first detection value is a value obtained by subtracting the correction value from the second detection value, and
the controller controls a process of, when a predetermined detection result is obtained by the second detector, calculating the first detection value.

3. The inhaler device according to claim 1 or 2, wherein the controller controls to switch a mode of the first detector to an operation mode to detect the reflected light or a sleep mode to stop detection of the reflected light, in accordance with a detection result obtained by the second detector.

4. The inhaler device according to claim 3, wherein
the inhaler device further comprises a lid capable of opening and closing an opening that communicates with the internal space of the container, and
the second detector detects opening and closing of the opening with the lid.

5. The inhaler device according to claim 4, wherein the controller controls to switch a mode of the first detector to the operation mode when opening of the opening with the lid is detected by the second detector and controls to switch the mode of the first detector to the sleep mode when closing of the opening with the lid is detected by the second detector.

6. The inhaler device according to claim 4 or 5, wherein the controller controls a process of calculating the correction value when opening of the opening with the lid is detected by the second detector.

7. The inhaler device according to claim 4 or 5, wherein
the controller controls a process of calculating the correction value and storing the calculated correction value in the memory when closing of the opening with the lid is detected by the second detector and controls a process of correcting the second detection value by using the correction value stored in the memory when the mode of the first detector is switched to the operation mode after the storage.

8. The inhaler device according to claim 3, wherein
the inhaler device further comprises a power supply that stores electric power, and
the second detector detects connection and cancellation of charging to the power supply by the user.

9. The inhaler device according to claim 8, wherein the controller controls to switch the mode of the first detector to the operation mode when cancellation of charging to the power supply is detected by the second detector and to switch the mode of the first detector to the sleep mode when connection of charging to the power supply is detected by the second detector.

10. The inhaler device according to claim 8 or 9, wherein the controller calculates the correction value when cancellation of charging to the power supply is detected by the second detector.

11. The inhaler device according to claim 8 or 9, wherein
the controller controls a process of calculating the correction value and storing the calculated correction value in the memory when connection of charging to the power supply is detected by the second detector and controls a process of correcting the second detection value by using the correction value stored in the memory when the mode of the first detector is switched to the operation mode after the storage.

12. The inhaler device according to any one of claims 1 to 11, wherein the controller determines whether the stick substrate is accommodated in the internal space in accordance with the first detection value.

13. The inhaler device according to any one of claims 1 to 11, wherein the controller performs a determination on a crud adhering to the container in accordance with the first detection value.

14. The inhaler device according to any one of claims 1 to 13, wherein the controller performs a determination on a state of the container in accordance with the first detection value and a predetermined threshold and corrects the threshold in accordance with a temporal change in the correction value.

15. A control method for an inhaler device, the control method being executed by a computer, the inhaler device including
a container capable of accommodating a stick substrate in an internal space;
a first detector that radiates light to the internal space and detects reflected light received;
a second detector that detects an action of a user to the inhaler device;
a memory that stores a correction value used to calculate a first detection value corresponding to an intensity of reflected light detected by the first detector by correcting a second detection value indicating an intensity of reflected light detected by the first detector, the control method comprising:
performing a determination on a state of the container in accordance with the first detection value; and
controlling a process of, when a predetermined detection result is obtained by the second detector, calculating the correction value and storing the correction value in the memory.
